# EUROPEAN PATENT APPLICATION

(11) **EP 1 308 718 A2**
(43) Date of publication of application: **07.05.2003**
(21) Application number: 02257585.6
(22) Date of filing: 01.11.2002
(51) Int. Cl.: G01N 25/28, G01N 25/32, A61L 2/20, A61L 2/26

(54) **Apparatus and method for monitoring of oxidative gas or vapor**

(30) Priority: 02.11.2001 US 16057
(71) Applicant: ETHICON, INC., Somerville, NJ 08876 (US)
(72) Inventor: Hui, Henry K., Laguna Niguel, CA 92677 (US); Engstrom, Keith, Laguna Niguel, CA 92677 (US); Fryer, Ben, Lake Forest, CA 92630 (US); Timm, Debra, Foothill Ranch, CA 92610 (US); Lin, Szu-Min, Laguna Hills, CA 92653 (US); Lemus, Anthony, Villa Park, CA 92861 (US)
(74) Representative: Fisher, Adrian John

(57) **Abstract**

An apparatus for monitoring the concentration of an oxidative gas or vapor includes a first thermocouple junction and a chemical substance coupled to the first thermocouple junction. The chemical substance is reactive with the oxidative gas or vapor to produce heat. The apparatus further includes a second thermocouple junction coupled in series to the first thermocouple junction. A net voltage is generated across the first and second thermocouple junctions upon exposure of the chemical substance to the oxidative gas or vapor. The net voltage corresponds to the concentration of the oxidative gas or vapor.

## Description

### Claim of Priority

This application is a continuation-in-part of U.S. Utility Patent Application No. 09/741,594 filed December 19, 2000 which is a continuation-in-part of U.S. Utility Patent Application No. 09/468,767 filed December 21, 1999, the disclosures of which are hereby incorporated in their entirety by reference herein.

### Field of the Invention

The invention relates to devices and techniques for monitoring the concentrations of an oxidative gas or vapor.

### Background of the Invention

Medical and surgical instruments have traditionally been sterilized using heat (e.g., exposure to steam), or chemical vapors (e.g., formaldehyde or ethylene oxide). However, both heat and chemical sterilizations have drawbacks. For example, many medical devices, such as fiberoptic devices, endoscopes, power tools, etc. are sensitive to heat, moisture, or both. Additionally, formaldehyde and ethylene oxide are both toxic gases which pose potential health risks to health workers. After sterilization with ethylene oxide, the sterilized articles require long aeration times to remove any remaining toxic material. This aeration step makes the sterilization cycle times undesirably long.

Sterilization using hydrogen peroxide vapor has been shown to have some advantages over other chemical sterilization processes (e.g., see U.S. Pat. Nos. 4,169,123 and 4,169,124). The combination of hydrogen peroxide vapor and a plasma provides additional advantages, as disclosed in U.S. Pat. No. 4,643,876. U.S. Pat. No. 4,756,882 discloses the use of hydrogen peroxide vapor, generated from an aqueous solution of hydrogen peroxide, as a precursor of the reactive species generated by a plasma. The combination of plasma and hydrogen peroxide vapor in close proximity with the sterilized articles acts to sterilize the articles.

Furthermore, use of low concentrations of hydrogen peroxide vapor has other advantages when used for chemical sterilization. Hydrogen peroxide is easy to handle, can be stored for long periods of time, is efficacious, and mixes readily with water. In addition, the products of decomposition of hydrogen peroxide are water and oxygen, which are both non-toxic.

However, there are problems with using hydrogen peroxide for sterilization. First, in order to be effective, devices must be exposed to a specified concentration of hydrogen peroxide. If the concentration of hydrogen peroxide is not sufficient, the article may require longer time and/or higher temperature to achieve sterilization. Second, if too much hydrogen peroxide is present, there is a risk of damaging the sterilized articles, particularly if they contain nylon, neoprene, or acrylic. For hydrogen peroxide absorbent materials, too much peroxide may leave an unacceptable residue on the sterilized article that may be incompatible with the user or patient. In addition, the use of too much hydrogen peroxide increases the cost of sterilization. Third, hydrogen peroxide concentration levels can decrease during the course of the sterilization process due to various factors, such as reactions with some surfaces which are undergoing sterilization, or permeation into and through some plastic materials. Fourth, hydrogen peroxide vapor can condense onto the walls of the sterilization chamber or onto equipment in the chamber, potentially degrading or harming the equipment. It is therefore important to be able to determine the concentration of hydrogen peroxide vapor in the sterilization chamber so that enough hydrogen peroxide is present to be effective, yet not so much that the sterilized articles or other equipment are damaged.

Furthermore, the concentration of hydrogen peroxide vapor can vary from one section of the sterilized articles to another. Even under equilibrium conditions, there may be regions of the sterilization chamber which are exposed to higher or lower concentrations of hydrogen peroxide due to restrictions of diffusion caused by other equipment in the chamber, or by the sterilized articles themselves. In particular, an enclosed volume with only a narrow opening will have a lower concentration of hydrogen peroxide than one with a wider opening. Under dynamic conditions (e.g., hydrogen peroxide is introduced into the chamber via an inlet port while at the same time, it is pumped out of an outlet port), the hydrogen peroxide concentration at a particular position in the chamber is a function of various factors, including the inlet flow, outlet pumping speed, and geometrical configuration of the system's inlet and outlet ports, sterilization chamber, and other equipment in the chamber, including the sterilized articles.

Various methods for determining hydrogen peroxide concentration levels in sterilization chambers have previously been disclosed. Ando et al. (U.S. Pat. No. 5,608,156) disclose using a semiconductor gas sensor as a means for measuring vapor phase hydrogen peroxide concentrations. The reaction time of the sensor is several tens of seconds, and the relation between the sensor output and the concentration of the hydrogen peroxide vapor varies with changes in pressure. Most hydrogen peroxide vapor sterilization procedures involve several treatment steps, usually including at least one step in vacuum. The response of the sensor to hydrogen peroxide through the treatment steps will therefore change, depending on the pressure used in each treatment step.

Cummings (U.S. Pat. No. 4,843,867) discloses a system for determining the concentration of hydrogen peroxide vapor *in situ* by simultaneous measurements of two separate properties, such as dew point and relative humidity. A microprocessor is then used to fit the two measurements into a model to calculate the hydrogen peroxide concentration. The method uses an indirect approximation based on a number of empirical assumptions, and the accuracy will vary depending on how closely the conditions in the sterilization chamber resemble those used to develop the model. This method also does not yield information concerning the differing concentrations of hydrogen peroxide at various positions within the sterilization chamber.

Van Den Berg et al. (U.S. Pat. No. 5,600,142) disclose a method of using near-infrared (NIR) spectroscopy to detect hydrogen peroxide vapor. Hydrogen peroxide has an absorption peak at about 1420 nm (nanometers) which can be used to determine its concentration. However, water is always present when hydrogen peroxide is present, since water is a decomposition product of hydrogen peroxide. Because water also absorbs near-infrared radiation at 1420 nm, it interferes with the determination of the hydrogen peroxide concentration. In order to correct for this interference, the water vapor concentration is determined separately by an absorption measurement at wavelengths which hydrogen peroxide does not absorb. This measured water vapor concentration is then used to correct the absorbance at 1420 nm for the contribution due to water. However, this correction measurement also suffers from contributions due to contaminants, such as various organic molecules, which absorb in the spectral region of the correction measurement. Since one does not normally know what organic molecules are present, the correction factor is therefore somewhat unreliable.

Furthermore, the NIR method requires absorption measurements at two different wavelengths and making corrections for the presence of water vapor, organic contaminants, or both. The electronic equipment for doing these corrections is complex and expensive, and the correction for the presence of organic compounds is subject to error. Additionally, the calculated hydrogen peroxide concentration is an average concentration over the volume which absorbs the near-infrared radiation, not a localized measurement of concentration at particular positions within the sterilization chamber.

United States Patent No. 4,783,317 discloses an apparatus for monitoring the concentration of hydrogen peroxide in liquid media, e.g. aqueous solutions for scrubbing the flue gases emanating from waste-incineration plants or large capacity firing systems. By exploiting the exothermic reaction of hydrogen peroxide with reducing agents (e.g. gaseous sulfur dioxide), the apparatus is able to measure the concentration of hydrogen peroxide in the liquid medium. The U-shaped apparatus comprises a thermally insulated measuring cell, a supply line which supplies a partial stream of the liquid from the source to the measuring cell, and a discharge line which returns the liquid to the source. In the measuring cell, the liquid is combined with a small stream of a reducing agent from a separate supply line, and the temperature of the mixture is monitored by a sensor. By comparing this temperature to the temperature of the liquid prior to entering the measuring cell, the apparatus measures temperature rise due to the ongoing exothermic reaction which is a function of the concentration of hydrogen peroxide in the liquid.

### Summary of the Invention

In one aspect, the present invention provides an apparatus for monitoring the concentration of an oxidative gas or vapor. The apparatus comprises a first thermocouple junction and a chemical substance coupled to the first thermocouple junction. The chemical substance is reactive with the oxidative gas or vapor to produce heat. The apparatus further comprises a second thermocouple junction coupled in series to the first thermocouple junction. A net voltage is generated across the first and second thermocouple junctions upon exposure of the chemical substance to the oxidative gas or vapor. The net voltage corresponds to the concentration of the oxidative gas or vapor.

In another aspect, the present invention provides a method of monitoring the concentration of an oxidative gas or vapor. The method comprises providing a first thermocouple junction and a second thermocouple junction coupled together in series. The first thermocouple junction is further coupled to a chemical substance which undergoes an exothermic reaction with the oxidative gas or vapor to be monitored. The method further comprises exposing the chemical substance to the oxidative gas or vapor, thereby generating a net voltage across the first and second thermocouple junctions. The net voltage is a function of the concentration of the oxidative gas or vapor. The method further comprises measuring the net voltage across the first and second thermocouple junctions as an indication of the oxidative gas or vapor.

### Brief Description of the Drawings

Figures 1A 1B, 1C, 1D, and 1E schematically illustrate various embodiments of a concentration monitor compatible with embodiments of the present invention and which comprise a carrier, a chemical substance, and a temperature probe.

Figure 2 schematically illustrates a sterilization system compatible with embodiments of the present invention.

Figures 3A, 3B, 3C, 3D, and 3E schematically illustrate various embodiments of a concentration monitor comprising a reference temperature probe compatible with embodiments of the present invention.

Figure 4A schematically illustrates a concentration monitor comprising an integrated circuit chip compatible with embodiments of the present invention.

Figure 4B schematically illustrates a concentration monitor comprising thermocouple junctions comprising thin conductive films compatible with embodiments of the present invention.

### Detailed Description of the Preferred Embodiment

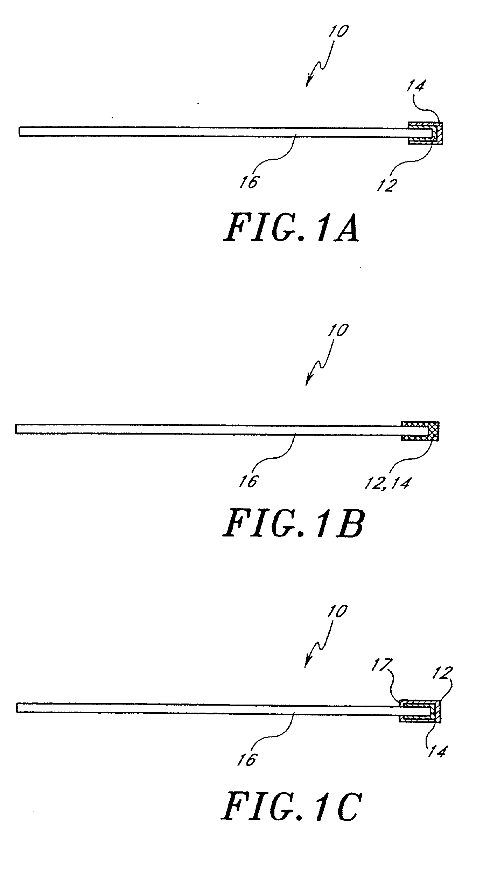

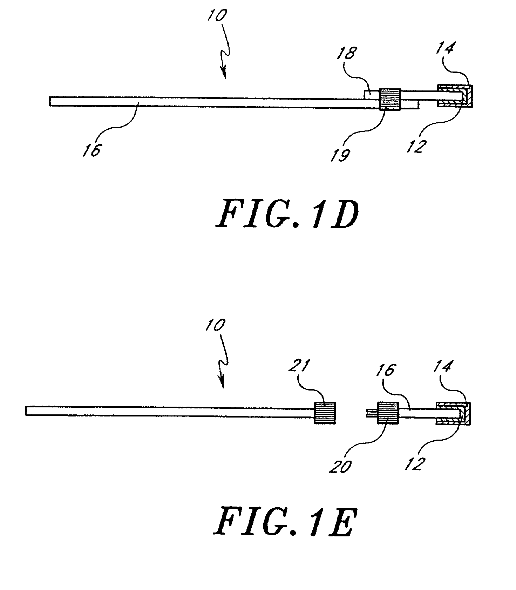

Figures 1A, 1B, 1C, 1D, and 1E illustrate embodiments of a concentration monitor 10 compatible with embodiments of the present invention. In certain embodiments of the present invention, a concentration monitor **10** comprises a carrier **12**, a chemical substance **14**, and a temperature probe **16**. All of the elements of the concentration monitor **10** must be compatible with its operating conditions. Concentration monitors **10** compatible with the present invention can operate under a wide range of pressures, such as atmospheric pressures or sub-atmospheric pressures (i.e., vacuum pressures). For use in a sterilization system utilizing hydrogen peroxide vapor with or without plasma, the carrier **12**, chemical substance **14**, and temperature probe **16** must all be compatible with operations under sterilization conditions and with exposure to hydrogen peroxide vapor and plasma. Persons skilled in the art recognize that there is a wide variety of materials and structures which can be selected as the carrier **12** in these embodiments. The carrier **12** couples the chemical substance **14** in close proximity to the temperature probe **16** so as to minimize the thermal losses between them. Examples of adequate carriers include, but are not limited to, acrylic, epoxy, nylons, polyurethane, polyhydroxyethylenemethacrylate (polyHEMA), polymethylmethacrylate (PMMA), polyvinylpyrrolidone (PVP), polyvinylalcohol (PVA), silicone, tape, or vacuum grease. Additionally, the carrier **12** can either be configured to expose the chemical substance **14** directly to the environment, or to enclose the chemical substance **14** in a gas permeable pouch, such as Tyvek tubing, or a gas impermeable enclosure with a hole or holes. In certain embodiments, the chemical substance **14** can be coupled directly to the temperature probe **16** without use of a carrier. For example, the chemical substance **14** can be formed as an integral part of the temperature probe **16** or, if the chemical substance **14** is sufficiently adhesive, it can be directly coupled to the temperature probe **16**. Chemical vapor deposition or electrochemical plating can also be used to couple the chemical substance **14** directly to the temperature probe **16**.

The chemical substance **14** undergoes an exothermic reaction with the oxidative gas or vapor to be monitored, producing a detectable amount of thermal energy (i.e., heat) upon exposure to the oxidative gas or vapor to be monitored. Persons skilled in the art are able to choose an appropriate chemical substance **14** which yields a sufficient amount of heat upon exposure to the relevant range of concentrations of the oxidative gas or vapor to be measured. Examples of chemical substances **14** for use in a hydrogen peroxide sterilization system include, but are not limited to, substances that catalytically decompose hydrogen peroxide, substances that are easily oxidized by hydrogen peroxide, and substances that contain hydroxyl functional groups. Substances that catalytically decompose hydrogen peroxide include, but are not limited to, catalase, copper and copper alloys, iron, silver, platinum, and palladium. Substances that are easily oxidized by hydrogen peroxide include, but are not limited to, magnesium chloride (MgCl₂), iron (II) compounds such as iron (II) acetate, potassium iodide (KI), sodium thiosulfate, and sulfides and disulfides such as molybdenum disulfide, 1,2-ethanedithiol, methyl disulfide, cysteine, methionine, and polysulfides. Substances that contain hydroxyl functional groups include, but are not limited to, polyethylene glycol (PEG), polyethylene oxide (PEO), and polyvinyl alcohol (PVA). These substances can be in the form of polymers that comprise hydroxyl functional groups, and persons skilled in the art appreciate that such polymers can also be co-polymers. In addition, a combination of these above-described substances may be chosen as the chemical substance **14**. Furthermore, persons skilled in the art are able to select the appropriate amount of chemical substance **14** to yield a sufficient amount of heat upon exposure to the relevant range of hydrogen peroxide concentrations.

Various configurations compatible with use with embodiments of the present invention are illustrated in Figures 1A, 1B, 1C, 1D, and 1E. Figure 1A shows a temperature probe **16** coated with a thin layer of carrier **12** on the tip of the probe **16** and the chemical substance **14** is coated on the outside of the carrier **12**. Figure 1B shows the chemical substance **14** is mixed with the carrier **12** and applied onto the tip of the temperature probe **16**. For example, a chemical substance **14** such as PEG is mixed with a carrier **12** such as acrylic binder in an aqueous suspension, then coated onto a temperature probe **16**. The chemical substance **14** is accessible for reaction as the hydrogen peroxide diffuses into the carrier. Figure 1C show the chemical substance **14** is enclosed onto the tip of the temperature probe **16** with a carrier **12**. The carrier **12** is a gas-permeable pouch with a heat-sealed area **17**, which typically is composed of a nonwoven polyolefin material, such as Tyvek® (nonwoven polyethelene) sold by E.I. du Pont de Nemours and Co. of Wilmington, Delaware or CSR (central supply room) wrapping material (nonwoven polypropylene) sold by Kimberly-Clark Corp. of Dallas, Texas. The carrier **12** can also be a gas-impermeable pouch or other enclosure with one or more holes to allow the diffusion of gas or vapor to react with the chemical substance **14** retained in the enclosure. Figure 1D shows a chemical substance **14** coupled to a heat-conducting material **18** with a carrier **12**, and the heat-conducting material **18** is coupled to the temperature probe **16** with a substrate **19**. The substrate **19** can be tape, adhesive, or any other coupling means. The heat-conducting material **18** can be metallic wire or any other materials which can properly conduct heat to the temperature probe **16**. Figure 1E shows a chemical substance **14** coupled to a temperature probe **16** with a carrier **12**, and two parts of the temperature probe **16** can be connected and disconnected with a male connector **20** and a female connector **21**.

The temperature probe **16** is a device which measures the temperature at a particular location. One embodiment of the present invention utilizes a fiberoptic temperature probe, such as a Luxtron® 3100 fluoroptic thermometer, as the temperature probe **16**. This fiberoptic temperature probe **16** is coated with Teflon and therefore is very compatible to any oxidative gas or vapor. Another embodiment utilizes a temperature probe **16** which is a thermocouple probe which utilizes a junction of two metals or alloys. The thermocouple junction produces a voltage which is a known function of the junction's temperature. Measurements of this voltage across the thermocouple junction can therefore be converted into measurements of the junction's temperature. Thermocouple junctions can be made quite small (e.g., by spot welding together two wires of 0.025-millimeter diameter composed of differing alloys), so they can be positioned into size-restricted volumes. In yet other embodiments, the temperature probe **16** can be a thermistor, glass thermometer, resistance temperature detector (RTD) probe, temperature strip, optical temperature sensor, or infrared temperature sensor.

Table 1 illustrates the increases of temperature measured by a concentration monitor **10** with potassium iodide (KI) as the chemical substance **14**. The tip of the fiberoptic temperature probe was first coated with a thin layer of Dow Corning high vacuum grease (part number 2021846-0888). About 0.15 grams of KI powder was then applied onto the vacuum grease. This configuration is the same as illustrated in Figure 1A. The measurements were conducted by suspending the concentration monitor **10** in a vacuum chamber heated to 45 °C, evacuating the chamber, recording the initial probe temperature, injecting hydrogen peroxide into the chamber, recording the temperature after all hydrogen peroxide was vaporized, evacuating the chamber to remove the hydrogen peroxide, and venting the chamber. The measurements were repeated with different concentrations of hydrogen peroxide injected into the chamber. The same temperature probe **16** was reused for all the measurements, and the results are shown in Table 1. As can be seen from Table 1, KI produces a measurable increase of temperature with increasing concentration of hydrogen peroxide. Additionally, this concentration monitor **10** can be reused many times.

**Table 1:**

| Concentration of H₂O₂ (mg/L) | Temperature increase (°C) |
|---|---|
| 0.2 | 3.0 |
| 0.4 | 8.3 |
| 0.8 | 19.2 |
| 1.3 | 24.2 |
| 2.1 | 33.7 |

Table 2 provides data on the measured temperature increases with varying concentrations of hydrogen peroxide for a concentration monitor **10** utilizing different chemical substances **14**. Same test conditions and probe configurations were used in these temperature measurements. As can be seen from Table 2, each of the chemical substances **14** produced a measurable temperature rise which increased with increasing hydrogen peroxide concentration.

**Table 2:**

| Chemical substance | Temperature increase (°C) | | |
|---|---|---|---|
| | 0.4 mg/L | 1.0 mg/L | 2.1 mg/L |
| Platinum on Alumina | 13.5 | 17.2 | --- |
| Catalase | 1.1 | --- | 6.9 |
| Iron (II) acetate | 62.5 | 83.1 | --- |
| Magnesium Chloride | 0.8 | --- | 4.4 |

The utility of using a thermocouple junction as the temperature probe **16** is illustrated in Table 3. For these measurements, the concentration monitor **10** was configured as illustrated in Figure 1A. The test conditions of Table 1 were also used for these measurements. Table 3 illustrates that significant temperature increases were also observed using a thermocouple temperature probe **16**.

**Table 3:**

| Concentration of H₂O₂ (mg/L) | Temperature increase (°C) |
|---|---|
| 0.2 | 2.7 |
| 0.4 | 11.9 |
| 0.8 | 19.3 |
| 2.1 | 24.2 |

The utility of using double-sided tape as the carrier **12** is illustrated by Table 4, which presents the temperature increases measured by a fiberoptic temperature probe **16**. A thin layer of 3M Scotch double-sided tape was first applied to the tip of the fiberoptic probe **16**. About 0.15 grams of KI powder was then coated onto the tape. Table 1 test conditions were repeated for these measurements. It is apparent from Table 4 that measurable increases of temperature were detected for increasing H₂O₂ concentration when using double-sided tape as the carrier **12**.

**Table 4:**

| Concentration of H₂O₂ (mg/L) | Temperature increase (°C) |
|---|---|
| 0.4 | 9.3 |
| 1 | 16.8 |
| 2.1 | 31.2 |

The utility of using epoxy as the carrier **12** is illustrated by Table 5, which presents the temperature increases measured by a fiberoptic temperature probe **16**. The concentration monitor **10** was constructed by applying a thin layer of Cole-Palmer 8778 epoxy on an aluminum wire. About 0.15 grams of KI powder was then applied and dried onto the epoxy. Finally, the aluminum wire was attached to the temperature probe **16**. Table 1 test conditions were repeated for these measurements. It is apparent that measurable increases of temperature were detected for increasing H₂O₂ concentration when using epoxy as the carrier **12**.

**Table 5:**

| Concentration of H₂O₂ (mg/L) | Temperature increase (°C) |
|---|---|
| 0.4 | 7.8 |
| 1 | 12.9 |
| 2.1 | 20.1 |

The utility of using an enclosure as the carrier **12** to enclose the chemical substance **14** is illustrated by Tables 6 and 7, which illustrate the increase of temperature detected by a fiberoptic temperature probe **16** with KI contained in an enclosure. For Table 6, the enclosure was PVC shrink tubing with holes. The holes were small enough to trap the KI powder but large enough to allow the diffusion of gas or vapor into the PVC tubing. For Table 7, the enclosure was gas-permeable Tyvek tubing fabricated from heat-sealed 1073B Tyvek. The inner diameter of the enclosure was about 0.5 centimeters, and its length was approximately 1.5 centimeters. For Table 6, about 0.2 grams of KI powder was enclosed in the PVC tubing and the concentration monitor **10** was re-used for all measurements. For Table 7, about 0.2 grams of KI powder was enclosed in the Tyvek pouch and the concentration monitor **10** was also re-used for all measurements. Table 1 test conditions were used for these measurements. It is apparent that measurable increases of temperature were detected for increasing H₂O₂ concentration when using both embodiments of a gas-permeable pouch as the carrier **12**. The results also demonstrate that the concentration monitor **10** can be re-used and the measurements are reproducible.

**Table 6:**

| Concentration of H₂O₂ (mg/L) | Temperature increase (°C) | | |
|---|---|---|---|
| | Trial #1 | Trial #2 | Average |
| 0.2 | 1.1 | 1.1 | 1.1 |
| 0.4 | 9.5 | 8.8 | 9.2 |
| 1.0 | 13.6 | 13.6 | 13.6 |

**Table 7:**

| Concentration of H₂O₂ (mg/L) | Temperature increase (°C) | | |
|---|---|---|---|
| | Trial #1 | Trial #2 | Average |
| 0.4 | 9.7 | 8.4 | 9.1 |
| 1.0 | 17.3 | 16.8 | 17.1 |
| 1.4 | 23.6 | 23.6 | 23.6 |

A chemical substance **14** comprising a polymer comprising hydroxyl functional groups may also be used to fabricate a hydrogen peroxide monitor. For example, polyethylene glycol or PEG, with a formulation of H(OCH₂CH₂)ₙOH, mixed with an acrylic binder in aqueous suspension provides a hydrogen peroxide monitor compatible with the present invention. Such chemical substances have a high specificity to oxidative gas or vapor, such as H₂O₂, and essentially no sensitivity to H₂O. Persons skilled in the art appreciate that other polymers containing hydroxyl functional groups are also compatible with the present invention.

To examine the utility of a PEG/acrylic suspension, various H₂O₂ monitors were fabricated using the following procedure. A 1:1 ratio by weight PEG/acrylic mixture was made by mixing and stirring 5 g of acrylic binder (Vivitone, Inc., product number 37-14125-001, metallic binder LNG) with 5 g of PEG (Aldrich, Inc., product number 30902-8, molecular weight of approximately 10,000) in a 20-g scintillation vial. Other embodiments compatible with the present invention can utilize ratios other than 1:1. The mixture was then heated to approximately 75 °C and stirred thoroughly. After allowing the mixture to cool to room temperature, the vial containing the suspension was capped and stored in a cool, dark environment.

To fabricate each H₂O₂ monitor, the metal surface of a thermocouple was chemically treated to improve the adhesion of the chemical substance **14** to the carrier **12**. The thermocouple was soaked in isopropyl alcohol for approximately two minutes and its end was brushed lightly to remove debris. After air-drying for approximately five minutes, the end of the thermocouple was soaked in approximately 10-20% by volume sulfuric acid (H₂SO₄) for approximately two minutes, then rinsed thoroughly in generous amounts of deionized water. The thermocouple was then dried in an oven at approximately 55 °C for approximately five minutes, then allowed to cool to room temperature outside the oven for approximately five minutes. The end of the thermocouple was then coated with the PEG/acrylic mixture by dipping the end of the thermocouple into the vial containing the mixture. Note that in order to produce a thicker overall coating, the end of the thermocouple can be dipped repeatedly. The thermocouple was then returned to the oven to dry at approximately 55 °C for approximately five minutes. A similar procedure was used to fabricate PEO/acrylic H₂O₂ monitors.

The above procedure can generate H₂O₂ monitors which are durable, inexpensive, and easy to manufacture. Also, PEG/acrylic mixtures have a relatively long shelf life of more than approximately three years. By utilizing a coating of the PEG/acrylic suspension, very small and flexible H₂O₂ monitors can be fabricated with different sizes and shapes. For example, if it is desirable to measure the H₂O₂ concentration within a narrow tube, the reactive chemical substance can be coated onto an optical fiber such as a Luxtron® fluoroptic temperature probe, a fiberoptic temperature probe, or on a metal wire of a thermistor or thermocouple assembly.

PEG/acrylic H₂O₂ monitors and PEO/acrylic H₂O₂ monitors fabricated by the above procedure were tested in a STERRAD® 100 low temperature, hydrogen peroxide gas plasma sterilization system. The sensitivity of these H₂O₂ monitors to hydrogen peroxide vapor is illustrated in Table 8 which provides the measured temperature increases in °C generated by the H₂O₂ monitors for different concentrations of H₂O₂ in the STERRAD® chamber. The change of temperature is referenced to the temperature read by the thermocouple just prior to the injection of H₂O₂.

**Table 8:**

| | Temperature Increase (°C) | |
|---|---|---|
| H₂O₂ (mg/L) | PEG/acrylic | PEO/acrylic |
| 0.41 | 2.6 | 2.0 |
| 0.77 | 3.4 | 3.5 |
| 1.45 | 5.8 | 5.6 |
| 2.87 | 9.4 | 9.7 |
| 5.73 | 16.1 | 14.0 |
| 11.5 | 24.2 | 22.0 |

Measured temperature increases for known H₂O₂ concentrations can be used to generate a calibration curve for such H₂O₂ monitors. The H₂O₂ responses of individual H₂O₂ monitors using the same chemical substance/carrier mixture were substantially similar to one another, indicating that H₂O₂ monitors with reproducible responses to H₂O₂ can be produced. For sufficient reproducibility among the H₂O₂ monitors using the same chemical substance/carrier mixture, a standard response equation can express the response for all such H₂O₂ monitors, thereby eliminating the need for individual calibration of the H₂O₂ monitors to convert the temperature change into a measurement of the H₂O₂ concentration.

H₂O₂ monitors compatible with the present invention with a reactive chemical substance/carrier such as the PEG/acrylic mixture can utilize other temperature probes **16** besides thermocouples. Appropriate temperature probes **16** include, but are not limited to, glass thermometers, thermocouples, thermistors, RTD probes, temperature strips, optical temperature sensors, and infrared temperature sensors. In addition, the sensing surface of the temperature probe **16** can be chemically or mechanically etched to improve the adhesion between the reactive chemical substance **14** and the temperature probe **16**. The reactive chemical substance **14** can be coated onto the temperature sensitive surface of the temperature probe **16** by a variety of methods, including but not limited to, dipping, painting, spraying, chemical vapor deposition, or electrochemical plating. For faster response times, it is preferable to apply a thin coat of the reactive chemical substance **14** on the temperature probe **16** with low thermal mass. The thickness of the coating can also be controlled by adjusting the dwelling time or the speed of withdrawal of the probe **16** from the solution as it is being coated, and the viscosity of the reactive chemical substance **14**. Additional layers of the reactive chemical substance **14** can be added to the initial coating to improve signal strength and/or sensitivity.

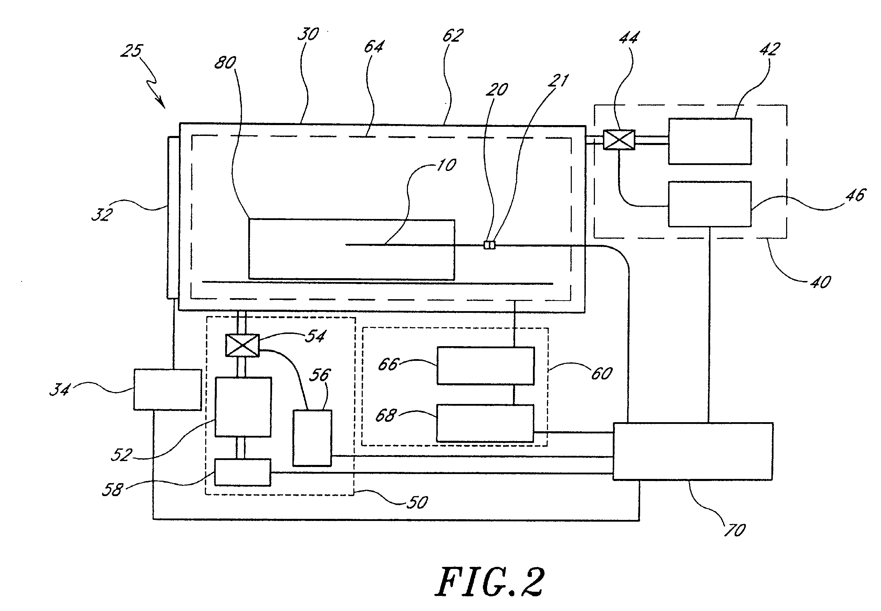

Figure 2 schematically illustrates a sterilization system **25** utilizing one embodiment of the present invention. The sterilization system **25** has a vacuum chamber **30** with a door **32** through which items to be sterilized are entered into and removed from the chamber **30**. The door is operated by utilizing a door controller **3**4. The vacuum chamber **30** also has a gas inlet system **40**, a gas outlet system **50**, and a radio-frequency (rf) system **60**. Other embodiments compatible with the present invention can utilize a low frequency plasma sterilization system, such as that described in "Sterilization System Employing Low Frequency Plasma", U.S. Patent Application No. 09/676,919, which is incorporated by reference herein. Comprising the gas inlet system **40** is a source of hydrogen peroxide (H₂O₂) **42**, a valve **44**, and a valve controller **46**. The gas outlet system **50** comprises a vacuum pumping system **52**, a valve **54**, a valve controller **56**, and a vacuum pumping system controller **58**. In order to apply radio-frequency energy to the H₂O₂ in the vacuum chamber **30**, the rf system **60** comprises a ground electrode **62**, a powered electrode **64**, a power source **66**, and a power controller **68**. The sterilization system **25** is operated by utilizing a control system **70** which receives input from the operator, and sends signals to the door controller **34**, valve controllers **46** and **56**, vacuum pumping system controller **58**, and power controller **68**. Coupled to the control system **70** (e.g., a microprocessor) is the concentration monitor **10**, which sends signals to the control system **70** which are converted into information about the H₂O₂ concentration in the vacuum chamber **30** at the location of the concentration monitor **10**. The sterilized article **80** is shown to be positioned in the chamber **30** with concentration monitor **10** located in the load region to monitor the concentration of hydrogen peroxide in the load region. Persons skilled in the art are able to select the appropriate devices to adequately practice the present invention.

The heat produced between the oxidative gas or vapor and the chemical substance **14** may not be the same for different configurations of the concentration monitor **10**, carrier **12**, and chemical substance **14**. Therefore, for a given type of concentration monitor **10**, a calibration curve needs to be established to determine the relationship between the concentration of oxidative gas or vapor and the heat produced. Once the calibration curve is established, the heat detected during the measurement can be converted to the concentration of the oxidative gas or vapor around the monitor **10**.

By coupling the operation of the sterilization system **25** with the H₂O₂ concentration measured by the concentration monitor **10**, the sterilization system **25** is assured of operating with an appropriate amount of H₂O₂ in the region of the articles to be sterilized. First, if the H₂O₂ concentration is determined to be too low for adequate sterilization, the control system **70** can signal the inlet valve controller **46** to open the inlet valve **44**, thereby permitting more H₂O₂ into the chamber **30**. Alternatively, if the H₂O₂ concentration is determined to be too high, the control system **70** can signal the outlet valve controller **56** to open the outlet valve **54**, thereby permitting the vacuum pumping system **52** to remove some H₂O₂ from the chamber **30**. Furthermore, if the sterilization system is being operated in a dynamic pumping mode (i.e., H₂O₂ is introduced into the chamber **30** via the inlet valve **44** while at the same time, it is pumped out via the outlet valve **54**), then either the inlet valve **44** or the outlet valve **54**, or both can be adjusted in response to the measured H₂O₂ concentration to ensure an appropriate level of H₂O₂.

Because the concentration monitor **10** provides localized information regarding the H₂O₂ concentration, it is important to correctly position the concentration monitor **10** within the sterilization chamber **30.** In some embodiments, the concentration monitor **10** is fixed to a particular position within the sterilization chamber **30** in proximity to the position of the sterilized articles **80**. In other embodiments, the concentration monitor **10** is not fixed to any particular position within the sterilization chamber **30**, but is placed on or near the sterilized article **80** itself. In this way, the concentration monitor **10** can be used to measure the H₂O₂ concentration to which the sterilized article **80** is exposed. In particular, if the sterilized article **80** has a region which is exposed to a reduced concentration of H₂O₂ due to occlusion or a reduced opening, then the concentration monitor **10** can be placed within this region to ensure that a sufficient H₂O₂ concentration is maintained to sterilize this region. The small size of the concentration monitor of the present invention permits the concentration monitor to be placed in very restricted volumes, such as the inner volume of a lumen, or in a container or wrapped tray. In still other embodiments of the present invention, a plurality of concentration monitors **10** can be utilized to measure the H₂O₂ concentration at various positions of interest.

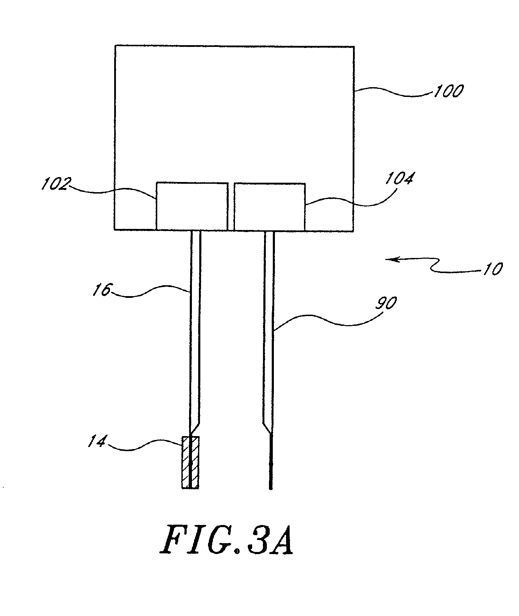

The temperature of the temperature probe **16** within the sterilization chamber **30** may fluctuate due to other factors unrelated to the hydrogen peroxide concentration. These non-H₂O₂-related temperature fluctuations may be misconstrued as resulting from changes of the H₂O₂ concentration in the sterilization chamber **30**, and may result in measurement errors. In certain embodiments, as schematically illustrated in Figure 3A, a reference temperature probe **90** can be utilized in conjunction with the temperature probe **16** of the concentration monitor **10** to provide a measure of the ambient temperature within the sterilization chamber **30** to improve the performance of the concentration monitor **10**.

The reference temperature probe **90** in proximity to the temperature probe **16** can then be used to measure the non-H₂O₂-related temperature fluctuations and compensate for these non-H₂O₂-related temperature fluctuations from the temperature reading of the temperature probe **16**. In certain embodiments, the non-H₂O₂-related temperature fluctuations are monitored substantially simultaneously with the temperature readings of the temperature probe **16**. Typically, the reference temperature probe **90** is substantially identical to the temperature probe **16**, but does not comprise the reactive chemical substance **14**. For example, a PEG/acrylic H₂O₂ concentration monitor **10** can comprise a reference temperature probe **90** with the acrylic binder but without the PEG polymer. Alternatively, the H₂O₂ concentration monitor **10** can comprise a bare reference temperature probe **90** without the binder or the reactive chemical substance **14**.

In the embodiment schematically illustrated in Figure 3A, the concentration monitor **10** comprises a reference temperature probe **90** and a temperature probe **16**, the reference temperature probe **90** separate from the temperature probe **16**. In certain such embodiments, the concentration monitor **10** comprises a microprocessor **100**, and the temperature probe **16** and the reference temperature probe **90** are each coupled to a separate data acquisition channel **102, 104** of the microprocessor **100**. The microprocessor **100** can comprise an algorithm, in hardware, software, or both, which subtracts the ambient temperature, as determined by the reference temperature probe **90**, from the temperature detected by the temperature probe **16** to arrive at the temperature rise due to the oxidative gas or vapor concentration in the sterilization chamber **30**. In such embodiments, electrical connections between the temperature probe **16**, reference temperature probe **90**, and microprocessor **100** require two data acquisition channels which, in certain embodiments, are too large in size to allow the temperature probe **16** and reference temperature probe **90** to be placed in certain narrow lumens.

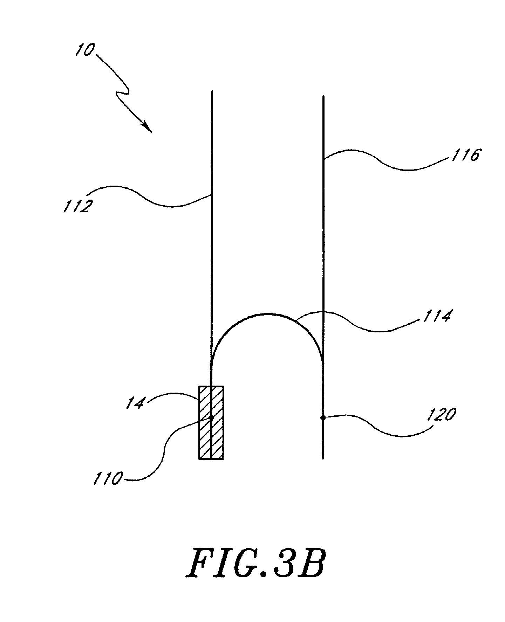

In certain embodiments, as schematically illustrated in Figure 3B, the concentration monitor **10** comprises a first thermocouple junction **110** and a chemical substance **14** coupled to the first thermocouple junction **110**. The chemical substance **14** is reactive with the oxidative gas or vapor to produce heat. The first thermocouple junction **110** comprises a first conductor **112** and a second conductor **114** coupled to the first conductor **112**, the second conductor **114** being different from the first conductor **112**.

The concentration monitor **10** further comprises a second thermocouple junction **120** which, in certain embodiments, is substantially similar to the first thermocouple junction **110**. The second thermocouple junction **120** is coupled in series to the first thermocouple junction **110.** In certain embodiments, as schematically illustrated in Figure 3B, the second thermocouple junction **120** comprises a third conductor **116** and the second conductor **114**, the third conductor **116** coupled to the second conductor **114**. In embodiments in which the second thermocouple junction **120** is substantially similar to the first thermocouple junction **110**, the third conductor **116** is substantially similar to the first conductor **112**. For example, the first conductor **112** and third conductor **116** can comprise constantan (copper-nickel alloy) wire and the second conductor **114** can comprise iron wire, thereby forming two J-type thermocouple junctions in series. Typically, such thermocouple junctions have sensitivities on the order of µV/°C. The first and second thermocouple junctions **110, 120** are substantially thermally isolated from one another, but are placed in the same diffusion-restricted region as one another.

Placed in an environment with no oxidative gas or vapor, the first thermocouple junction **110** and second thermocouple junction **120** each generates a voltage indicative of the ambient temperature. In embodiments in which the second thermocouple junction **120** is substantially similar to the first thermocouple junction **110**, both thermocouple junctions **110, 120** generate the same voltage but are oriented to have opposite polarity such that the net voltage across both the first thermocouple junction **110** and the second thermocouple junction **120** is zero. Such a concentration monitor **10** in an environment with no oxidative gas or vapor responds to temperature fluctuations by maintaining a zero net voltage across the two thermocouple junctions **110, 120**.

Upon exposing the chemical substance **14** to the oxidative gas or vapor, the heat generated by the chemical substance **14** increases the temperature of the first thermocouple junction **110** while the temperature of the second thermocouple junction **120** remains substantially unaffected, remaining at the ambient temperature. In embodiments in which the second thermocouple junction **120** is substantially similar to the first thermocouple junction **110,** the voltage generated by the first thermocouple junction **110** is different from the voltage generated by the second thermocouple junction **120** in the presence of the oxidative gas or vapor. The net voltage across the first and second thermocouple junctions **110, 120** is responsive to the temperature difference between the first thermocouple junction **110** with the chemical substance **14** and the second thermocouple junction **120** without the chemical substance **14**. Since any temperature fluctuations not due to the oxidative gas or vapor concentration affect both thermocouple junctions **110, 120** equally, the net voltage across both the first thermocouple junction **110** and second thermocouple junction **120** then corresponds to the concentration of the oxidative gas or vapor.

In certain embodiments, the first thermocouple junction **110** and second thermocouple junction **120** are each formed by welding together two conductors comprising different materials. Alternatively, one or both of the thermocouple junctions **110, 120** is formed by twisting together two conductors comprising different materials. Other embodiments compatible with the present invention can form the first and second thermocouple junctions **110, 120** by connecting the two conductors together using other methods. As schematically illustrated in Figures 3A and 3B, the conductors of certain embodiments are metal wires. The materials for the conductors which comprise the first thermocouple junction **110** and second thermocouple junction **120** are selected to provide thermocouple junctions with sufficient thermoelectric sensitivity and generally low cost, high electrical conductivity, low thermal conductivity, and good material compatibility with the sterilization process.

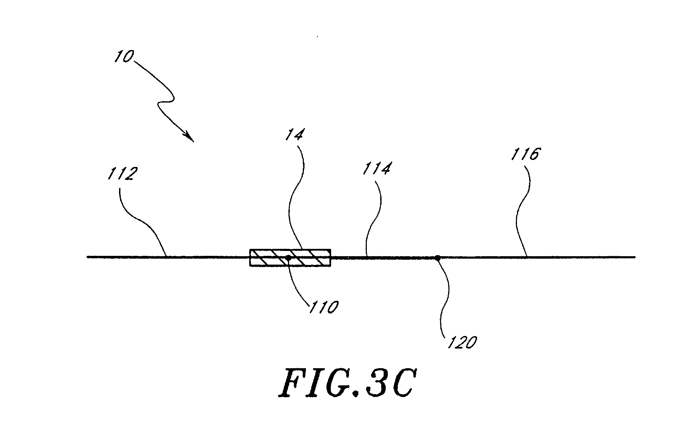

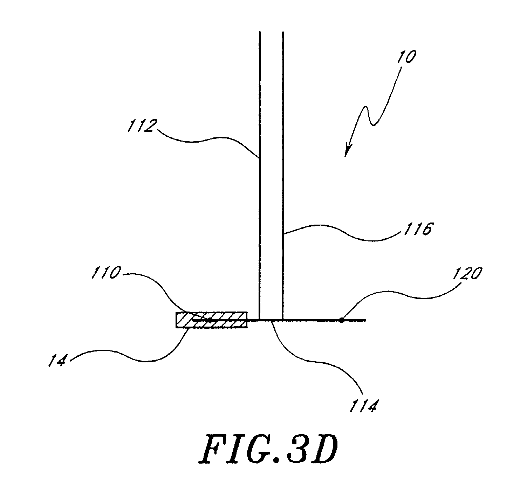

As schematically illustrated in Figure 3C, in certain embodiments, the concentration monitor **10** has a linear configuration and comprises a first thermocouple junction **110** and a second thermocouple junction **120**. The first thermocouple junction **110** is formed by coupling a first conductor **112** to a second conductor **114** such that the first conductor **112** and second conductor **114** are substantially colinear. The second thermocouple junction **120** is formed by coupling the second conductor **114** to a third conductor **116** such that the second conductor **114** and third conductor **116** are also substantially colinear. The first thermocouple junction **110** is coupled to the chemical substance **14** and the second thermocouple junction **120** is not coupled to the chemical substance **14**. Such an embodiment is especially useful for monitoring the concentration of the oxidative gas or vapor within a long, narrow lumen. Similarly, in the embodiment schematically illustrated in Figure 3D, the concentration monitor **10** has a "T" configuration. Other configurations are compatible with embodiments of the present invention, and the particular embodiment utilized can be designed for compatibility with the region in which the oxidative gas or vapor concentration is to be measured.

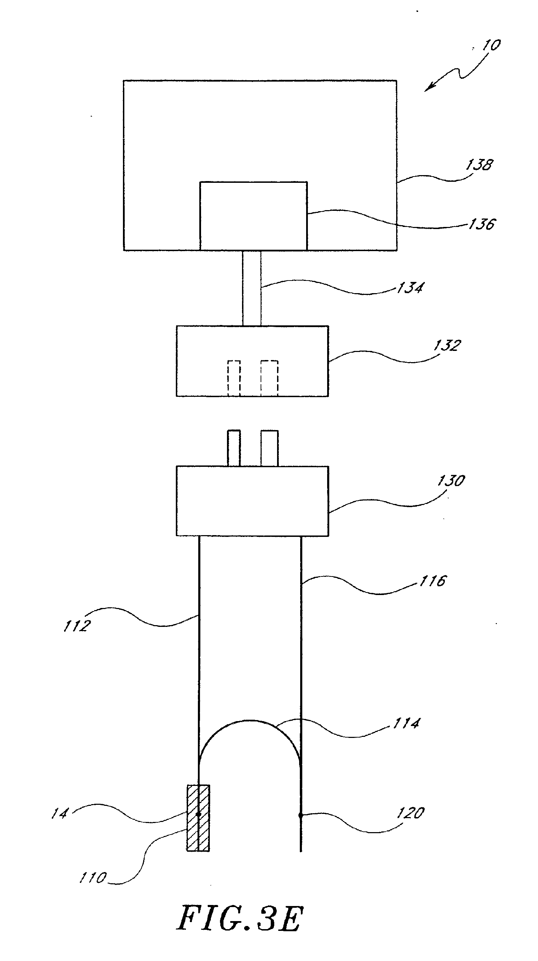

As schematically illustrated in Figure 3E, in certain embodiments, the concentration monitor **10** comprises a first connector **130**, second connector **132**, cable **134**, data acquisition channel **136**, and microprocessor **138**. The first connector **130** and second connector **132** can be coupled together to electrically connect the first conductor **112** and third conductor **116** via the cable **134** to the data acquisition channel **136** of the microprocessor **138**. The first connector **130** and second connector **132** can also be decoupled so that, for example, the concentration monitor **10** can be repositioned at a different location within the sterilization chamber.

The embodiments schematically illustrated in Figures 3B-3E provide advantages over the embodiment schematically illustrated in Figure 3A. First, using two thermocouple junctions **110, 120** in series requires only one sensing circuit or one data acquisition channel to monitor the concentration of the oxidative gas or vapor, as opposed to two data acquisition channels as in Figure 3A. Besides providing a potential cost savings, using only one data acquisition channel or sensing circuit eliminates the potential effects of variations between the multiple channels or sensing circuits. Second, since the net voltage across the two thermocouple junctions **110, 120** represents a temperature difference rather than an absolute temperature, the dynamic range of values is smaller, so the an analog-to-digital converter with a given number of bits can thereby provide greater precision when used in the chemical concentration measuring system. Third, because only one pair of conductors is needed to detect the net voltage across the two thermocouple junctions **110, 120**, the size of the concentration monitor **10** can be made smaller to fit into various diffusion-restricted environments, such as narrow lumens.

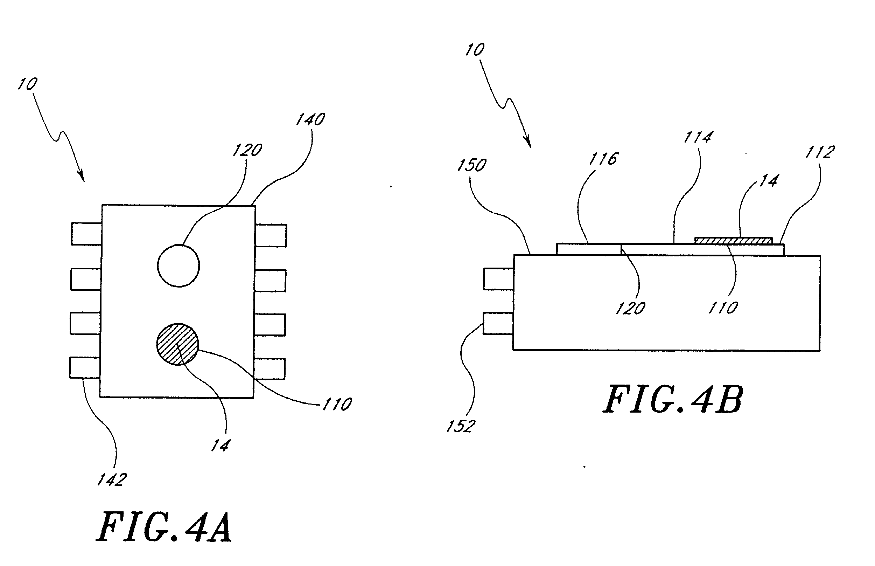

As schematically illustrated in Figure 4A, in certain embodiments, the concentration monitor **10** comprises an integrated circuit chip **140** which comprises circuitry which includes the first and second thermocouple junctions **110, 120**, chemical substance **14**, and a microprocessor or other sensing circuit (not shown). The integrated circuit chip **140** is configured to output a signal on one or more of its pins **142** to communicate the measured concentration to the rest of the chemical concentration measuring system. In certain embodiments, standard lithographic techniques can be used to fabricate the first and second thermocouple junctions **110, 120** by depositing and etching overlapping metal layers with different materials onto a substrate. Persons skilled in the art are able to fabricate such concentration monitors **10** in accordance with embodiments of the present invention.

As schematically illustrated in Figure 4B, in certain embodiments, the first and second thermocouple junctions **110, 120** are formed from a first conductor **112**, second conductor **114**, and third conductor **116**, where one or more of the conductors comprises a thin conductive film configuration. The chemical substance **14** is coupled to the first thermocouple junction **110**, and in certain embodiments, can also have a thin film configuration. In embodiments in which first and second thermocouple junctions **110**, **120** formed by thin film conductors are part of a thin film concentration monitor **150**, a signal indicative of the measured concentration can be provided on one or more of the pins **152**. In certain embodiments, a thin film concentration monitor **150** may be incorporated into the packaging of the articles to be sterilized, thereby providing localized concentration information from a plurality of articles in the load.

In embodiments in which the first thermocouple junction **110** is substantially similar to the second thermocouple junction **120**, further advantages are achieved. First, the concentration monitor **10** does not require an algorithm to correct for ambient temperature, since the net voltage across the two thermocouple junctions due to ambient temperature is null. Second, a cold junction compensation is not required, since ambient temperature has effectively no contribution. Third, only a relatively small amount of the second conductor **114** is needed to form the two thermocouple junctions, thereby realizing a cost savings over other embodiments.

This invention may be embodied in other specific forms without departing from the essential characteristics as described herein. The embodiments described above are to be considered in all respects as illustrative only and not restrictive in any manner. The scope of the invention is indicated by the following claims rather than by the foregoing description. Any and all changes which come within the meaning and range of equivalency of the claims are to be considered within their scope.

## Claims

1. An apparatus for monitoring the concentration of an oxidative gas or vapor, the apparatus comprising:
a first thermocouple junction;
a chemical substance coupled to the first thermocouple junction, the chemical substance reactive with the oxidative gas or vapor to produce heat; and
a second thermocouple junction coupled in series to the first thermocouple junction, whereby a net voltage is generated across the first and second thermocouple junctions upon exposure of the chemical substance to the oxidative gas or vapor, the net voltage corresponding to the concentration of the oxidative gas or vapor.

2. The apparatus as defined in Claim 1, wherein the second thermocouple junction is substantially similar to the first thermocouple junction.

3. The apparatus as defined in Claim 2, wherein the net voltage across the first and second thermocouple junctions is zero when the chemical substance is not exposed to the oxidative gas or vapor.

4. The apparatus as defined in Claim 1, wherein the oxidative gas or vapor comprises hydrogen peroxide.

5. The apparatus as defined in Claim 1, wherein the chemical substance is a material that chemically reacts with hydrogen peroxide.

6. The apparatus as defined in Claim 1, wherein the chemical substance is a material that catalytically decomposes hydrogen peroxide.

7. The apparatus as defined in Claim 1, wherein the chemical substance is a material that is oxidized by hydrogen peroxide.

8. The apparatus as defined in Claim 1, wherein the chemical substance comprises hydroxyl functional groups.

9. The apparatus as defined in Claim 1, wherein the apparatus further comprises a carrier which couples the chemical substance to the first thermocouple junction.

10. The apparatus as defined in Claim 1, wherein the apparatus further comprising a heat conductor between the chemical substance and the first thermocouple junction.

11. The apparatus as defined in Claim 1, wherein the apparatus further comprises a connector to connect and disconnect a first portion of the apparatus coupled to the chemical substance to a remaining portion of the apparatus.

12. The apparatus as defined in Claim 1, wherein the apparatus is positionable at one or more locations, whereby the net voltage is a function of the concentration of the oxidative gas or vapor at the location.

13. The apparatus as defined in Claim 1, wherein the second thermocouple junction is in a diffusion-restricted region with the first thermocouple junction.

14. The apparatus as defined in Claim 1, wherein the apparatus further comprises an integrated circuit chip which comprises the first thermocouple junction and second thermocouple junction.

15. The apparatus as defined in Claim 1, wherein the first thermocouple junction comprises a first conductor and a second conductor coupled to the first conductor, the second conductor being different from the first conductor, and the second thermocouple junction comprises the second conductor coupled to a third conductor.

16. The apparatus as defined in Claim 15, wherein the third conductor is composed of the same material as the first conductor.

17. The apparatus of Claim 15, wherein at least one of the first conductor, second conductor, and third conductor comprises a conductive film.

18. A method of monitoring the concentration of an oxidative gas or vapor, the method comprising:
providing a first thermocouple junction and a second thermocouple junction coupled together in series, the first thermocouple junction coupled to a chemical substance which undergoes an exothermic reaction with the oxidative gas or vapor to be monitored;
exposing the chemical substance to the oxidative gas or vapor, thereby generating a net voltage across the first and second thermocouple junctions, whereby the net voltage is a function of the concentration of the oxidative gas or vapor;
measuring the net voltage across the first and second thermocouple junctions as an indication of the concentration of the oxidative gas or vapor.

19. The method as defined in Claim 18, wherein the net voltage across the first and second thermocouple junctions is zero when the chemical substance is not exposed to the oxidative gas or vapor.

20. The method as defined in Claim 18, wherein the oxidative gas or vapor comprises hydrogen peroxide.

21. The method as defined in Claim 18, wherein the chemical substance is a material that chemically reacts with hydrogen peroxide.

22. The method as defined in Claim 18, wherein the chemical substance is a material that catalytically decomposes hydrogen peroxide.

23. The method as defined in Claim 18, wherein the chemical substance is a material that is oxidized by hydrogen peroxide.

24. The method as defined in Claim 18, wherein the chemical substance comprises hydroxyl functional groups.

25. The method as defined in Claim 18, wherein the chemical substance is coupled to the first thermocouple junction by a carrier.

26. The method as defined in Claim 25, wherein the carrier comprises a gas-permeable pouch or gas-impermeable enclosure with at least one hole.

27. The method as defined in Claim 18, additionally comprising moving the apparatus to one or more locations, whereby the net voltage is a function of the concentration of the oxidative gas or vapor at the location.

28. A sterilization system operated by a user, wherein the sterilization system comprises:
a chamber;
a door in the chamber;
a source of oxidative gas or vapor in fluid connection with the chamber;
a chemical concentration measuring system comprising at least one apparatus according to Claim 1; and
a control system which receives input from the chemical concentration measuring system to produce a desired concentration of said oxidative gas or vapor.

29. The system as defined in Claim 28, wherein the system further comprises a pumping system to reduce the pressure in the chamber.
